(19) Europäisches Patentamt
European Patent Office
Office européen des brevets

(11) Publication number: 0 294 071 A2

# (12) EUROPEAN PATENT APPLICATION

(21) Application number: 88304669.0

(22) Date of filing: 23.05.88

(51) Int. Cl.4: A61K 39/29 , A61K 47/00

(30) Priority: 03.06.87 US 57195

(43) Date of publication of application: 07.12.88 Bulletin 88/49

(84) Designated Contracting States: CH DE FR GB IT LI NL

(71) Applicant: MERCK & CO. INC.
126, East Lincoln Avenue P.O. Box 2000
Rahway New Jersey 07065-0900(US)

(72) Inventor: McAleer, William J.
717 Marietta Drive
Ambler, PA 19002(US)
Inventor: Schaefer, Ted F.
22 Woodstock Circle
Collegeville, PA 19426(US)
Inventor: Lehman, E. Dale
104 Crestwood Drive
Lansdale, PA 19446(US)

(74) Representative: Hesketh, Alan, Dr.
European Patent Department Merck & Co., Inc. Terlings Park Eastwick Road
Harlow Essex, CM20 2QR(GB)

## (54) Hepatitis B vaccines made with pre-formed aluminum hydroxide gels, and processes thereof.

(57) Hepatitis B antigen is stably adsorbed to a pre-formed aluminum hydroxide gel.

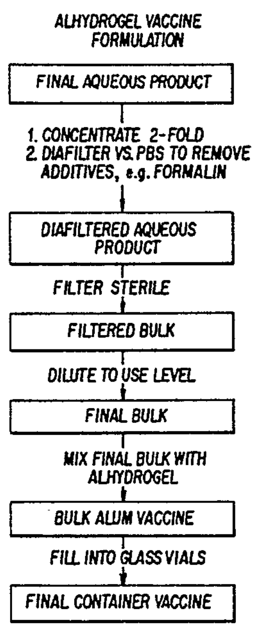


FIG. 1

EP 0 294 071 A2

# HEPATITIS B VACCINES MADE WITH PRE-FORMED ALUMINUM HYDROXIDE GELS, AND PROCESSES THEREOF

## INTRODUCTION

Hepatitis B surface antigen often occurs as a glycoprotein-protein complex that confers immunity against the pathological effects of subsequent infection by hepatitis B virus. Sources for safe, rapid and inexpensive manufacture of the antigen for vaccination purposes have been limited to serum or plasma from patients who synthesize large quantities of the requisite antigen, usually in the form of 22 nm particles. With the advent of recombinant DNA techniques, DNA coding for the surface antigen is inserted into yeast, E. coli or other cellular systems, then expressed. The resulting polypeptide product varies substantially with the DNA construction used as well as the host or cellular systems employed to express the inserted DNA.

The HB virion is composed of two groups of structural proteins, the core proteins and the envelope or surface ("S") proteins. In addition to being the major surface proteins of the virion, i.e., Dane particle, the "S" proteins are the sole constituents of Australia antigen, or 22 nm particles. The "S" proteins are the translational products of a large open reading frame (ORF) encoding 389-400 amino acids, depending upon serotype. This ORF is demarcated into three domains, each of which begins with an ATG codon that is capable of functioning as a translational initiation site in vivo. These domains are referred to as preS-1 (108-119 amino acids) pres 2 (55 amino acids), and S (226 amino acids) in their respective 5′-3′ order in the gene. The six protein products derived from this ORF have the following compositions:

1) gp42 (42,000 dalton glycoprotein) = preS-1/S-2/S (meaning preS-1 contiguous with preS-2, continuous with S),

2) p39 (p = protein) = preS-1/S-2/S,

3) gp36 = preS-2/S (two glycosylation sites),

4) gp33 = preS-2/S (one glycosylation site),

5) gp27 = S (one glycosylation site),

6) p24 = S.

All six proteins are present to an approximately equimolar extent in the HBV Dane particle. In the 22 nm particle, the 4 smaller proteins are present to an approximately equimolar extent, while gp42 and p39 are present in at most one or a few molecules per particle. The preS-1 and pres-2 regions may have functions of promoting secretion of the S region. For reviews of these fundamental properties of the protein, see Tiollais, P. et al., Science 213, 406 (1981) and Milich, D. R. et al., Proc. Natl. Acad. Sci. 82, 8168 (1985).

The preS-2 region of the hepatitis B antigen comprises about 55AA residues. Its presence provides a dominant epitope that is more immunogenic in vivo than epitopes of the S protein, according to . Neurath, A. R. et al., Science 224, 392 (1984), Neurath, A. R. et al., Nature 315, 154 (1985), and Milich, D. R. et al., supra. The preS-2 polypeptide also has receptor-like properties for polymerized human serum albumin (pHSA), a trait also possessed by liver cells which are known to bind pHSA, Machida, A. et al., Gastroenterology 86, 910 (1984).

Since the presence of preS-2 sequences in the surface antigen is a desirable property for the purposes of immunization, expression systems have been developed for the expression of preS-1/S-2/S protein and other variants.

One purpose of the present invention is to formulate hepatitis B vaccines that are stable for long periods of storage. The present invention is directed to hepatitis B vaccines having adjuvant gels that are stably adsorbed to purified surface antigen. The length of time for stable adsorption is at least twenty months without substantial leaching, elution or deadsorption of the antigen from the adjuvant gel. No other commercially marketed hepatitis vaccine has this property of stability.

Another purpose of the present invention is to eliminate a variety of steps in the vaccine manufacturing process. For example, admixture of a pre-formed adjuvant gel with the hepatitis surface antigen is employed instead of liquid admixture of adjuvant and antigen before precipitation, gelling or emulsification. As a consequence, applicants have eliminated a variety of standard steps typically performed after admixing liquid adjuvant and antigen. Accordingly, the present process does not require any of the following steps:
autoclaving, formalin removal, thimerosal addition to the final aqueous product, or use of expensive glass-lined tanks.

Preparation of vaccines having as antigens novel recombinant proteins frequently requires new methods or novel combinations of old methods, since the composition of the source for recombinant forms, e.g.,

yeast, are different from conventional sources, e.g., serum. Expression of recombinant proteins requires novel uses of translational machinery. One cannot predict what methods are useful for preparing recombinant vaccines derived from recombinant proteins expressed in recombinant cell cultures on the basis of the knowledge and know-how of methods useful for preparing conventional vaccines derived from classical or otherwise conventional sources. Purification processes designed for preparing vaccines require unusual purity in the product, another indication of the unpredicability in the art. For a similar view, see U.S. Patent 4,624,918.

## BRIEF DESCRIPTION OF THE INVENTION

A process is disclosed for preparing a hepatitis B vaccine having antigen stably adsorbed to a preformed aluminum hydroxide gel comprising the steps of

(a) treating purified hepatitis B surface antigen, or protein thereof, with formalin;

(b) removing the formalin;

(c) sterile filtering;

(d) adding buffer to an appropriate use level; and

(e) adsorbing the product of step (d) with a preformed, stable, viscous, homogeneous and non-pyrogenic aluminum hydroxide gel, yielding a vaccine comprising a sterile hepatitis B surface antigen, or portion thereof, that is stably adsorbed to a preformed aluminum hydroxide gel.

Hepatitis vaccines produced by this process are also encompassed.

## DETAILED DESCRIPTION OF THE INVENTION

The processes of the present invention involve new methods of preparing recombinant vaccines containing hepatitis B surface antigen from yeast extracts. Purified recombinant hepatitis B surface antigen is adsorbed to pre-formed gels of aluminum hydroxide. This single step produces an unusually stable vaccine with additional savings from fewer manipulations.

It will be understood that the novel preparation processes of the present invention are applicable to a range of expressed recombinant hepatitis B proteins. The processes of the present invention are designed to provide rapid and efficient methods of preparing vaccines containing variants of hepatitis B surface antigens. For example, conservative substitutions in the preS-1/S-2/S amino acid sequence generally will not result in any substantial or novel modification of the principles and practice of the present invention. Conservative substitutions are known; see Elfassi, E. et al., J. Theor. Biol. 121, 371 (1986). Alternatively, deletions within the S region or at the carboxy terminal end of the S region will not in general require any modifications of the processes for preparing vaccines discussed herein. It will be understood that recombinant hepatitis surface antigen in this application includes any such variations or portions of the amino acid sequence, whether by conservative amino acid substitution, deletion, or other process, provided that the resulting surface antigen or portion thereof, is immunochemically reactive with antibodies specific for the preS-1/S-2/S protein, the 22 nm particle, Australia antigen, or other natural form of the hepatitis surface antigen sequence.

Many yeast expression systems are clearly adequate for providing sources of the recombinant surface antigen. The S. cerevisiae strain 2150-2-3 transformed with pHBS56-GAP 347/33 in Example I is intended as a merely incidental source. Other yeast vectors include but are not limited to shuttle vectors, cosmid plasmids, chimeric plasmids, and those having sequences derived from 2-micron circle plasmids. A variety of promoters and other yeast transcriptional control sequences may be used to express the inserted DNA sequence coding for surface antigen, including those of GAL10 and the alpha mating factor.

The genus Saccharomyces is composed of a variety of species. Most commonly used is Saccharomyces cerevisiae, or baker's yeast, as a host for the recombinant DNA-mediated expression of a variety of foreign polypeptides. However, the distinctions among other species of the genus Saccharomyces are not always well-defined. Many of these species are capable of interbreeding with S. cerevisiae and are likely to possess regulatable promoters which are analogous or identical to promoters in S. cerevisiae, including but not limited to GAL10, ADH2, and/or alpha mating factor promoters. Therefore, it will be obvious to those skilled in the art that, for the expression of pre-S-containing polypeptides, the selection of a host strain extends to other species of the genus Saccharomyces, including but not limited to carlsbergensis, uvarum, rouxii, montanus, kluyveri, elongisporus, norbensis, oviformis, and diastaticus.

Several yeast genera, such as Hansenula, Candida, Torulopsis, and Pichia, have been shown to contain

similar metabolic pathways for the utilization of methanol as a sole carbon source for growth. The gene for alcohol oxidase, an enzyme which participates in this metabolic pathway, has been isolated from Pichia pastoris. The P. pastoris alcohol oxidase promoter has been isolated and shown to be susceptible to methanol induction of expression. Such an inducible promoter is useful for the expression of polypeptides which are negatively selected in yeast. In particular, this promoter has been shown to be active on a plasmid for the inducible expression of the S domain in P. pastoris in particulate form. This observation highlights the ability of other yeast genera to function as hosts for the recombinant DNA-mediated expression of S polypeptides in immunologically active form. Therefore, it will be apparent to those skilled in the art that, for the expression of surface antigen polypeptides such as the preS-1/S-2/S protein, the selection of a host strain extends to species from other genera of yeast from the Families Saccharomycetaceae and Cryptococcaceae, including, but not limited to Pichia, Candida, Hansenula, Torulopsis, Kluyveromyces, and Saccharomycopsis.

Purification of recombinant surface antigen to a final aqueous product (Fig. 1) was performed by the methods of Howard, C.R. et al., J. Vir. Meth. 14, 25 (1986). Yeast cells transformed by a yeast vector expressing surface antigen of Hepatitis B virus were grown and harvested. A cell paste was disrupted, and protease inhibitors added to reduce undesired hydrolysis of the surface antigen. The resulting yeast cell extract was treated with detergents and/or denaturants to extract the surface antigen. After various steps of concentration, dialysis or diafiltration, the detergent was removed.

The desired antigen was then adsorbed onto a solid-phase adsorbent, then eluted. Hydrophobic affinity chromatography followed, then the surface antigen was eluted with detergent after initial washing out of undesired proteinaceous contaminants. The resulting product was concentrated, sterile filtered, subjected to molecular sieve chromatography, and finally treated to convert intrachain disulphide-bonded peptides to intrachain disulphide-bonded particles.

The resulting aqueous particle mixture was dialyzed against PBS, diluted in PBS to a concentration of about 20 mcg/mL, and sterile filtered. The product was labeled sterilized antigen solution.

In preparing the sterilized antigen solution, various protease inhibitors may be used at different stages of the purification process. Suitable protease inhibitors include but are not limited to metal-chelating agents, heavy metal ions, SH blocking reagents, substrate-like compounds of proteases, polypeptides that inhibit proteases, and the like. The preferred inhibitor is phenylmethyl sulfonyl fluoride (PMSF) at about 2mM in final concentration.

Extraction of surface antigen in the yeast cell extract may require one or more detergents and/or denaturants. Applicants prefer about 0.1% (w/v) Triton X-100, but it will be apparent that other reagents are suitable. A variety of neutral or nonionic detergents can be used, including but not limited to detergents of the Triton N series, Triton X series, BRIJ series, Tween series or Emasol series, deoxycholate, octylglucopyranoside, or Nonidet NP-40. Zwitterionic detergents such as CHAPS or CHAPSO are also useful and suitable agents. Suitable protein denaturants include the generic class of compounds of the formula:

$$\begin{array}{c} \phantom{R-}X\phantom{-}H\phantom{-Y} \\ R-C-N-Y \end{array}$$

wherein

R is amino, loweralkylthio, loweralkyloxy, or sulfur;

X is amino, sulfur or oxygen; and

Y is hydrogen or amino.

Applicants have found that 0.1% (w/v) Triton X-100 is sufficient for extraction.

It will be readily apparent that the procedures and protocols for preparing sterilized antigen solution of this invention may be modified or varied by, for example, one or more additional steps involving hydrophobic chromatography, solid-phase chromatography, precipitation with salts such as ammonium sulfate, ion exchange chromatography, immunoaffinity chromatography or any other typical, ordinary and conventional technique useful for the purpose of recombinant protein purification.

Once the sterilized antigen solution is made, dilution in the appropriate buffer may be necessary to obtain a use level of antigen in final bulk form for admixture with preformed adjuvant gel. Generally, the only step required in preparing Final Bulk from the Final Aqueous Product (See Fig. 1) is a simple dilution. Applicants prefer to add additional steps, including treatment with formalin or other agent designed to inactivate viruses, and addition of thimerosol or other antibiotics. Most preferably, applicants prefer the steps of

(a) treating 100 volumes of the sterilized antigen solution (concentration = about 20 mcg/mL) with 1 volume of formalin at a dilution of about 1:40,

(b) adding, for each 200 volumes of resterilized, formalized antigen solution, one volume of 1% thimerosal, yielding the final aqueous product;

(c) concentration the final aqueous product;

(d) diafiltering against phosphate buffered saline to remove formalin, to yield the diafiltered aqueous product;

(e) sterile filtering to yield filtered bulk;

(f) diluting to use level to yield final bulk;

(g) mixing final bulk with Alhydrogel, which is a preformed adjuvant gel, to yield bulk alum vaccine; and

(h) filling glass vials with the bulk alum vaccine, to form the final container vaccine.

Formalin can be removed by other means, including dialysis or the addition of sodium bisulfite.

One principle of the present invention is the use of a preformed adjuvant gel in step (h), supra, for adsorption in a manner that provides substantially stable adsorption of surface antigen to the gel over a period lasting at least twenty months. The preformed characteristic of the adjuvant eliminates a variety of steps frequently employed after mixing antigen with adjuvant in the commercial production of vaccine, including removal of formalin, three to four cycles of settling and decanting, centrifugation to remove by products of the alum forming reaction, addition of bacteriocidal or bacteriostatic agent(s) such as thimerosal. Since thimerosal requires expensive glass lined tanks, its elimination makes the product cheaper to produce.

The conditions for adding the preformed adjuvant gel, and variations thereof are as follows.

To about nineteen volumes of hepatitis B surface antigen, 20-25 mcg/mL, is added about 1 volume of sterile Alhydrogel, Superfos a/s, Grade B [3% aluminum hydroxide gel], after which the mixture is stirred for about 1 hour at 20-23 °C. It is then stored at 2-8 °C until aliquoting into final containers.

The preformed adjuvant may be any preformed, stable, viscous, homogeneous and non-pyrogenic aluminum hydroxide gel that forms stable complexes with the purified antigen. The most preferred gel is Alhydrogel, a commercial product of Superfos A/S of Denmark. Other gels within the scope of the present invention include RESORPTAR®II and F-5000, both of Armour.

Aluminum hydroxide gels are to be distinguished from alum, the classic adjuvant. Alum is double salt of sulfate with the general formula $M'_2SO_4 \bullet M'''_2 - (SO_4)_3 \bullet 24H_2O$ or $M' M''♂(SO_4)_2\text{-}12H_2O$, wherein $M'$ is a monovalent metal and $M''$♂ is aluminum or other trivalent metal.

Specific discussions of the properties of Alhydrogel may be found in Jolles, P. et al., "Chemical and Biological Basis of Adjuvants", Molecular Biology, Biochemistry and Biophysics, Volume 13, pp. 112 et seq (1973); Herbert, W.J., "Mineral-oil adjuvants and the Immunization of Laboratory Animals" in Weir, D.M. (ed.) Handbook of Experimental Immunology, Vol. 3, pp. A3.11 et seq. Blackwell 1978; , "Alhydrogel: Aluminum Hydro xide Gel", commercial circular of E.M. Sargeant Pulp and Chemical Co., Inc., 6 Chelsea Road, Clifton, New Jersey 07012, pp 1-12, and references cited therein.

## EXAMPLE 1

### Expression of HBsAg in S. cerevisiae

Construction of yeast vectors carrying S gene sequences, as well as expression of S genes in transformed S. cerevisiae host cells were conducted according to Valenzuela, P. et al. Nature 298, 347 (1982) and McAleer, W.J. et al Nature 307, 178 (1984). The S. cerevisiae host cells employed in this invention for expression purposes are strain 2150-2-3, and the yeast vector pHBS56 GAP347/33 carries a glyceraldehyde-3 phosphate dehydrogenase promoter instead of the alcohol dehydrogenase promoter.

Cells were grown according to Wampler, D.E. et al Proc. Natl. Acad. Sci. 82, 6830 (1985), and harvested by continuous-flow centrifugation in a Sharples AS16 centrifuge (4 inch bowl, flow rate 3 L/minute). The cell paste was suspended in an equal volume of hypertonic phosphate buffer (0.1 M sodium phosphate, pH 7.2, 0.5 M NaCl). Phenylmethylsulphonyl fluoride (0.2 M in isopropanol) was added to a final concentration of 2 mM and the cells were disrupted by multiple passes through a high pressure

homogenizer (Gaulin Corp.) to yield a yeast cell extract.

The surface antigen synthesized in yeast is particulate and cell associated. After disruption of the cells, the antigen particles measure 17-20 nm in diameter, slightly smaller than the native particles found in the plasma of infected persons but otherwise of similar morphology.

The UV absorption pattern is identical to the plasma antigen with an El% of 45. SDS-PAGE under reducing conditions reveals a single predominant polypeptide of mol. wt. 25,000 corresponding to the non-glycosylated polypeptide I of HBsAg.

## EXAMPLE 2

### Purification of HBsAg

The resulting yeast cell extract was diluted with 4 vol. of 0.01 M phosphate buffer, pH 7.5, containing 0.1% Triton X-100 (Rohm & Haas, Inc). Cell debris was removed by continuous-flow centrifugation as before, the supernatant solution was concentrated 5-fold in a hollow fibre unit (H10X100, Amicon Corp.) and "diafiltered" with 5 vol. of phosphate-buffered saline [PBS (7mM sodium phosphate, pH 7.2, 0.15 M NaCl)]. Triton X-100 was removed using Amberlite XAD 4 beads (Rohm & Haas, Inc) and the solution clarified by centrifugation for 35 minutes at 9,000 x g and 4° C.

HBSAg was partially purified by adsorption and elution from fused silica (Aerosil 380, Degussa Corp.) and was routinely stored at -70° C. An insoluble precipitate that formed on thawing was removed by centrifugation at 8,000 x g for 45 minutes.

The HBsAg particles in the clarified solution were adsorbed onto a column of butyl Sepharose 4B (Pharmacia) which had been equilibrated with 50 mM MOPS (3-[N Morphilino]propanesulfonic acid) buffer, pH 7.0. Unadsorbed proteins were eluted with the same buffer and HBsAg then eluted with 0.1% Triton X-100 in 50 mM MOPS, pH 7.0. Triton X-100 was removed with Amberlite XAD-2 (Rohm & Haas), concentrated by ultrafiltration (XM-100 membrane, Amicon) and filtered through a 0.22 µm pore size membrane (Millex G V, Millipore). The HBsAg was further purified by gel filtration on Sepharose 6B (Pharmacia) equilibrated in PBS.

HBsAg of yeast origin consists of subunits which form particles via non-covalent hydrophobic interactions. These were converted to particles of interchain disulphide-bonded peptides as follows. The HBsAg solution was adjusted to 3 M potassium thiocyanate by mixing with an equal volume of 6M potassium thiocyanate in PBS and stirring for 16 hours at +4° C. Removal of potassium thiocyanate was then accomplished by diafiltration against 12 vol. of PBS. The antigen solution was diluted to 20 mcg/mL in PBS, then sterilized by filtering through a 0.2 µm membrane. The sterilized antigen solution was stored at 2-4° C.

## EXAMPLE 3

### Preparation and Formulation of Alhydrogel adsorbed vaccine

The sterilized antigen solution was reacted with formalin by adding, with mixing, 1/100th volume of a 1:40 dilution of formalin in distilled water. The mixture was placed in a 37° C incubator and stirred with a magnetic base. After 24 hours the material was aseptically transferred to a clean sterile bottle and placed again on the magnetic base. Incubation at 37° C was continued for an additional 48 hours, after which the solution was removed from the incubator and 1/200th volume of 1% Thimerosal was added. The solution was labeled Final Aqueous Product and stored at 2-8° C.

One liter of Final Aqueous Product containing 20 mg of protein was concentrated to 500 mL with an Amicon Spiral Cartridge, S1Y100, and the formalin and Thimerosal were removed by diafiltering with 6.6 mM sodium phosphate, pH 7.2, 0.15 M NaCl (phosphate buffer). After being sterilized by filtration the

solution, which contained 15.6 mg of protein in 538 mL, was diluted to the use level of 22 mcg/mL by adding 170 mL of phosphate buffer. The resulting solution was the Final Bulk form of the vaccine.

Alhydrogel adsorbed vaccine was prepared by adding, at 20-23° C, 12.5 mL of aluminum hydroxide gel [Alhydrogel, Superfos a/s, grade B, 3% $Al(OH)_3$] to 250 mL of final bulk solution containing 5.50 mg of protein, yielding 0.48 mg/ml of trivalent aluminum in the bulk alum vaccine. The mixture was stirred for 60 minutes and then stored at 2-8° C. Greater than 99% of the protein was adsorbed to the aluminum hydroxide gel as determined by radioimmune assay of the supernatant medium for HBsAg (AUSRIA II, Abbott).

Additional data on adsorption of antigen by Alhydrogel and its stability and maintenance is as follows:

(a) hepatitis B surface antigen remains >99% adsorbed to Alhydrogel for at least 20 months after initial adsorption, and,

(b) the vaccine is as potent after 15 months of storage time as when first formulated.

## EXAMPLE 4

### Effect of Adsorption Method on $ED_{50}$ and Relative Potency

Effective dosage ($ED_{50}$) is the dose in mcg/mL required to seroconvert 50% of the mice to seropositive for antibodies to HBsAg and relative potency (R.P.) is the $ED_{50}$ of test vaccine divided by $ED_{50}$ of Merck hepatitis B standard vaccine, Lot 815-1. Merck colony mice or Balb/c mice were inoculated intraperitoneally with 1.0 mL of serial two-fold dilutions of vaccine and bled four (Merck mice) or six (Balb/c mice) weeks later. Antibodies to HBsAg were determined with the "AUSAB-EIA" Diagnostic Kit (Abbott, Chicago, Ill.) and data were analyzed to determine seroconversion to different doses of vaccine. A probit analysis was performed, plots of empirical probit versus dose were made, iterative maximum liklihood least squares performed on the data and $ED_{50}$ (mcg/mL) values were obtained.

The preparation of vaccine lots is described in the following paragraphs.

To one liter aliquots of formalin-treated purified recombinant yeast-derived hepatitis B surface antigen (Final Aqueous Product, 20 mcg/mL protein), was added 85 mL of a 5.44% solution of aluminum potassium sulfate and the mixtures were immediately neutralized with 41 ml of N/1 NaOH. They were stirred for 10 minutes, the pH adjusted to pH 6.8 and then they were stirred two hours longer. The mixtures were labelled Alum Product 89404 or Alum Product 89426.

The Alum Products were divided into four 280 mL aliquots and labelled as one of the following:

(a) 89426-1
(b) 89426-2
(c) 89404-3
(d) 89404-4
(e) 89426-5
(f) 89426-6
(g) 89404-7
(h) 89404-8

Lots 89426-1, 89404-3, 89426-5 and 89404-7 were processed by the settle/decant method. Each 280 mL aliquot was allowed to separate by gravity into a lower alum precipitate phase and an upper supernatant phase. The upper phases were decanted and the alum precipitates were resuspended in either sterile 0.9% sodium chloride solution (saline; Lots 89426-1 and 89404-3) or sterile 0.9% sodium chloride solution buffered with 6.6mM sodium phosphate, pH 7.2 (PBS; Lots 89426-5 and 89404-7) to yield a final volume of 350 mL. The resuspended mixtures were stirred for 15 minutes and then allowed to stand at 20-23° C for two hours so that the alum settled to the bottom of the vessel. The supernatant fluids of each lot were withdrawn and the alum precipitates treated as above, except they were allowed to settle out overnight at 4° C. The settle/decant process was repeated two more times at 20-23° C. After the final decantation each alum precipitate was resuspended in 200 mL of saline or PBS, stirred for 30 minutes and then all the volumes adjusted to 250 mL with the appropriate menstrum. To each vaccine was added 1.25 mL of a 1% solution of Thimerosal (anti-microbial preservative), and the vaccines were stirred for one hour and then stored at 2-8° C.

Lots 89426-2, 89404-4, 89426-6 and 89404-8 were made by the centrifugation method. Each 280 mL aliquot was centrifuged at 3600 rpm, 10 minutes, 4° C in a Beckman JS-4.2 rotor causing the mixtures to separate into a supernatant liquid phase and a lower alum precipitate phase. The supernatant phases were decanted and the precipitates were resuspended in an amount of sterile saline (Lots 89426-2 and 89404-4) or PBS (Lots 89426-6 and 89404-8) to make a final volume of 250 ML. After mixing for twenty minutes, centrifuging and decanting as before, each alum precipitate was resuspended in sufficient menstruum to yield 200 mL of volume. Stirring was continued for one hour, after which the volumes were adjusted to 250 mL with the appropriate menstruum. Each vaccine was placed at +4° C, stirred for 18 hours and finally stored at the same temperature.

Lots 89426-9 and 89404-10 were prepared by the Alhydrogel method. One liter aliquots of formalin-treated purified recombinant yeast-derived hepatitis B surface antigen (Final Aqueous Product, 20 mcg/mL protein) were individually concentrated 2-fold with an Amicon concentration/dialysis cell using a $S1Y^{100}$ spiral wound cartridge. The Concentrated Final Aqueous Product was diafiltered with 6 liters of PBS to remove formalin and sterilized by filtering through a sterile Millipore Millex GV membrane, 0.2 micron pore size. The antigen solution was diluted to 22 mcg/mL with PBS and to 250 mL of the solution was added 12.5 mL of Alhydrogel (3% $Al[OH]_3$, Grade B, Superfos) after which it was stirred 70 minutes at 23° C. Radioimmune assay of the supernatant fluid showed that >99.9% of HBsAg was adsorbed onto Alhydrogel.

Note that, because fewer manipulations are involved in making vaccine by the Alhydrogel method, the anti-microbial preservative, Thimerosel, was omitted. Thimerosal contains mercury and its use should be avoided if possible.

Analysis of both the $ED_{50}$'s and the relative potencies (See Table below) showed that Alhydrogel vaccine had the highest relative potency and was statistically more potent than any of the other vaccines. Additionally, hepatitis B surface antigen remains >99% adsorbed to Alhydrogel for at least 20 months and the vaccine is as potent after storage for 15 months as when first formulated.

TABLE

| | | | Merck Mice | | Balb/c Mice | |
|---|---|---|---|---|---|---|
| | Adsorption | | | Relative | | Relative |
| Lot No. | Method | Menstruum | $ED_{50}$ | Potency | $ED_{50}$ | Potency |
| 89426-1 | Settle; Decant | Saline[1] | 1.87 | 0.27 | 0.92 | 0.41 |
| 89404-3 | Settle; Decant | Saline | 1.52 | 0.46 | 0.52 | 0.65 |
| 89421-2 | Centrifuge | Saline | 1.76 | 0.29 | 1.48 | 0.25 |
| 89404-4 | Centrifuge | Saline | 1.94 | 0.36 | 0.63 | 0.54 |
| 89426-5 | Settle; Decant | PBS[2] | 1.46 | 0.35 | 0.80 | 0.47 |
| 89404-4 | Settle; Decant | PBS | 1.20 | 0.59 | 0.44 | 0.78 |
| 89426-6 | Centrifuge | PBS | 1.25 | 0.40 | 0.64 | 0.59 |
| 89404-8 | Centrifuge | PBS | 1.68 | 0.42 | 0.37 | 0.91 |
| 89426-9 | Alhydrogel | PBS | 1.12 | 0.45 | 0.68 | 0.55 |
| 89404-10 | Alhydrogel | PBS | 1.01 | 0.70 | 0.39 | 0.86 |

[1] 0.9% Sodium Chloride Solution

[2] 0.9% Sodium Chloride Solution, 6.6mM Sodium Phosphate, pH 7.2. (PBS=Phosphate Buffered Saline)

While the foregoing specification teaches the principles of the present invention, with examples provided for the purpose of illustration, it will be understood that the practice of the invention encompasses all of the usual variations, adaptations, modifications, deletions or additions of procedures and protocols described herein, as come within the scope of the following claims.

## Claims

1. A hepatitis B vaccine comprising a preformed, stable, viscous, homogeneous and non-pyrogenic aluminum hydroxide gel that is stably adsorbed to surface antigen, or portion thereof, admixed thereto with said gel, said adsorption remaining substantially stable for at least 20 months.

2. The hepatitis B vaccine of Claim 1 wherein said gel is Alhydrogel.

3. A process for preparing a hepatitis B vaccine having antigen stably adsorbed to a preformed aluminum hydroxide gel comprising the steps of

(a) treating purified hepatitis B surface antigen, or portion thereof, with formalin;
(b) removing the formalin;
(c) sterile filtering;
(d) adding buffer to an appropriate use level; and

(e) adsorbing the product of step (d) with a preformed, stable, viscous, homogeneous and non-pyrogenic aluminum hydroxide gel, yielding a vaccine comprising a sterile hepatitis B surface antigen, or portion thereof, that is stably adsorbed to a preformed aluminum hydroxide gel.

4. The process of Claim 3 wherein said gel is Alhydrogel.

5. A process for preparing a bulk alum hepatitis B vaccine having antigen stably adsorbed to a preformed aluminum hydroxide gel comprising the steps of:

(a) diluting purified, sterilized hepatitis B surface antigen to a use level wherein the concentration of said antigen in buffer is between about 2.5µg/ml and 1.0 mg/ml;

(b) adding sufficient quantities of preformed aluminum hydroxide gel to yield, in a bulk alum vaccine, a final concentration of trivalent aluminum in the range of between about 0.3 mg/ml and 0.6 mg/ml, said gel being a preformed, stable, viscous, homogeneous and non-pyrogenic aluminum hydroxide gel, yielding the bulk alum vaccine comprising sterile recombinant B surface antigen, or portion thereof, that is stably adsorbed for at least 20 months to said gel.

6. The process of Claim 5, wherein the dilution of step (a) is performed to yield a use level of about 20 µg/ml of said antigen in buffer.

7. The process of Claim 5 or 6 wherein the final concentration of said gel in step (b) is about 0.48 mg/ml of trivalent aluminum in the bulk alum vaccine.

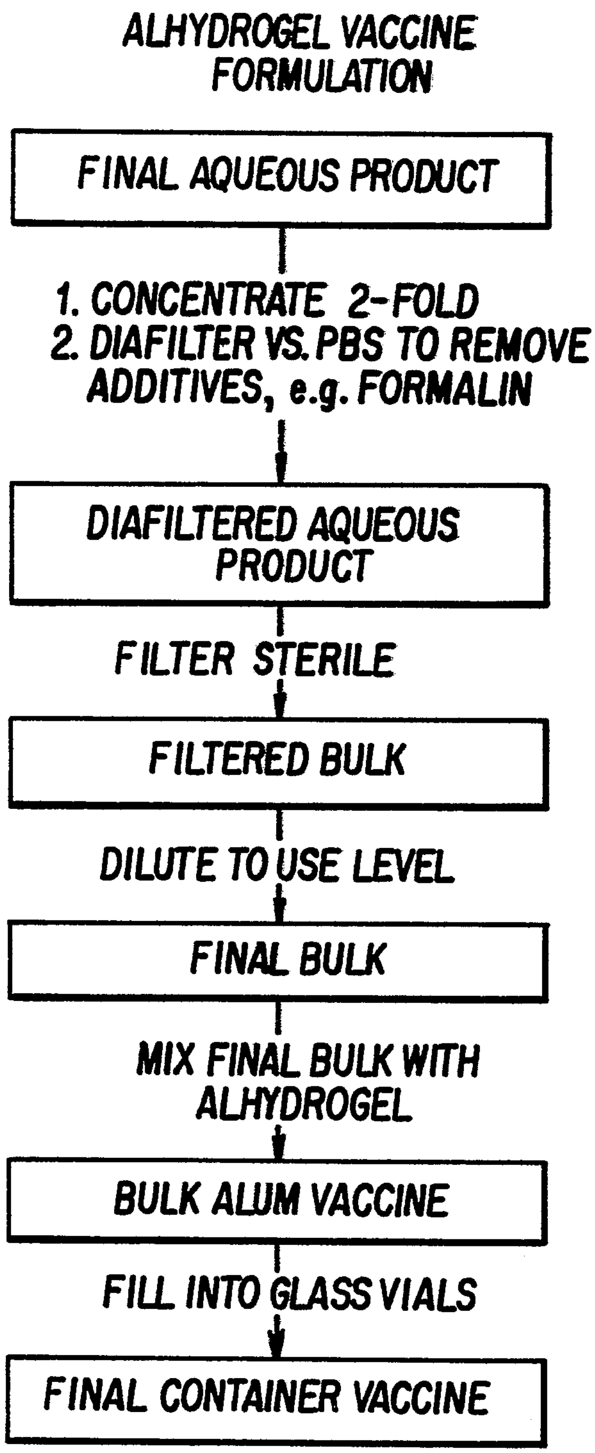
ALHYDROGEL VACCINE FORMULATION
FINAL AQUEOUS PRODUCT
1. CONCENTRATE 2-FOLD
2. DIAFILTER VS. PBS TO REMOVE ADDITIVES, e.g. FORMALIN
DIAFILTERED AQUEOUS PRODUCT
FILTER STERILE
FILTERED BULK
DILUTE TO USE LEVEL
FINAL BULK
MIX FINAL BULK WITH ALHYDROGEL
BULK ALUM VACCINE
FILL INTO GLASS VIALS
FINAL CONTAINER VACCINE

FIG. 1